# FASCICULE DE BREVET EUROPEEN

(11) **EP 1 066 264 B1**
(45) Date de publication et mention de la délivrance du brevet: **17.11.2004**
(21) Numéro de dépôt: 00900643.8
(22) Date de dépôt: 21.01.2000
(51) Int. Cl.: C07D 233/60, C07D 233/56, C07D 213/20, C07D 295/08, C07D 295/14, A61K 7/13

(54) **2-SULFONYLAMINOPHENOLS CATIONIQUES, LEUR UTILISATION A TITRE DE COUPLEUR POUR LA TEINTURE D'OXYDATION, COMPOSITIONS LES COMPRENANT, ET PROCEDES DE TEINTURE**
KATIONISCHE 2-SULFONYLAMINOPHENOLE, IHRE ANWENDUNG ALS KUPPLER FÜR DIE OXIDATIONSFÄRBUNG, DIESE ENTHALTENDE ZUSAMMENSETZUNGEN UND FÄRBUNGSVERFAHREN
NOVEL CATIONIC 2-SULPHONYLAMINOPHENOLS, THEIR USE AS COUPLERS FOR OXIDATION DYEING, COMPOSITIONS CONTAINING THEM AND DYEING METHODS

(30) Priorité: 21.01.1999 FR 9900640
(43) Date de publication de la demande: 10.01.2001
(73) Titulaire: L'OREAL, 75008 Paris (FR)
(72) Inventeur: VIDAL, Laurent, F-75013 Paris (FR); SAUNIER, Jean-Baptise, F-75014 Paris (FR)
(74) Mandataire: Miszputen, Laurent
(86) Numéro de dépôt international: PCT/FR2000/000142
(87) Numéro de publication internationale: WO 2000/042971

(56) Documents cités:
- FR-A- 2 196 997
- FR-A- 2 541 999
- FR-A- 2 586 913
- US-A- 4 975 092
- DATABASE CHEMABS [en ligne] CHEMICAL ABSTRACTS SERVICE, COLUMBUS, OHIO, US; STN, CAPLUS accession no. 1994:178093, XP002140690 -& CHEMICAL ABSTRACTS, vol. 120, no. 14, 4 avril 1994 (1994-04-04) Columbus, Ohio, US; abstract no. 178093s, XP002141029 & JP 05 150392 A (KONISHIROKU PHOTO IND)

## Description

L'invention a pour objet de nouveaux 2-sulfonylaminophénols cationiques de formule (I) comportant un groupement cationique, leur utilisation à titre de coupleur pour la teinture d'oxydation des fibres kératiniques et en particulier des fibres kératiniques humaines telles que les cheveux, les compositions de teinture d'oxydation les contenant en association avec au moins une base d'oxydation, et les procédés de teinture d'oxydation les mettant en oeuvre.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Pour obtenir des nuances rouges, on utilise généralement, seul ou en mélange avec d'autres bases, et en association avec des coupleurs appropriés, du 4-aminophénol, et pour obtenir des nuances bleues, on fait habituellement appel à des paraphénylènediamines. L'utilisation de coupleurs dérivés de métaphénylènediamines, en association avec des dérivés de paraphénylènediamines, conduit habituellement à des nuances bleues de solidité généralement médiocre.

Or la demanderesse vient maintenant de découvrir , de façon totalement inattendue et surprenante, que nouveaux 2-suifonylaminophénols de formule (I) définie ci-après comportant au moins un groupement cationique , non seulement conviennent pour une utilisation comme coupleur, mais en outre qu'ils permettent d'obtenir des compositions tinctoriales conduisant à des colorations puissantes, dans une large palette de couleurs, et présentant d'excellentes propriétés de résistances aux différents traitements que peuvent subir les fibres kératiniques.

Ces découvertes sont à la base de la présente invention.

l'invention a donc pour premier objet de nouveaux 2-sulfonylaminophénols de formule (I): 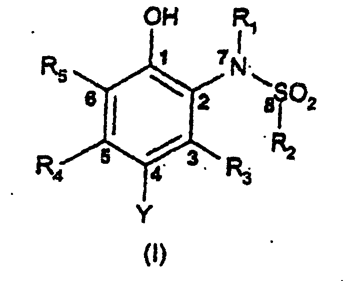 dans laquelle :
- R₁ représente un atome d'hydrogène ;
- R₂ représente un radical méthyle, éthyle ou diméthylamino ;
- R₃ représente un atome d'hydrogène ou un radical méthyle ;
- R₄ représente -NH-E-D₃, -NH(CO)-D₃, -NH(CO)-E-D₃, -NH(CO)O-E-D₃, -NH(CO)NH-E-D₃, ou -NH(SO₂)-E₃-D₃;
- R₅ représente un atome d'hydrogène, de chlore ou de fluor, ou un groupement méthyle, méthoxy, ou méthylamino ;
- Y représente un atome d'hydrogène ou de chlore, ou un groupement méthoxy, ou -OCH₂(CO)OCH₃ ;
- E représente un bras -(CH₂)_{q}-, q étant un nombre entier égal à 1 ou 2,
- D₃ est choisi parmi les groupements 3-méthylimidazolinium-1-yl, 3-(2-hydroxyéthyl)-imidazolinium-1-yl, 1,2,4-triazolium-1-yl, 1,2,4-triazolium-4-yl, N-alkyl(C₁-C₄)pyridin-2-yl-ium; N-alkyl(C₁-C₄)pyridin-3-yl-ium; N-alkyl(C₁-C₄)pyridin-4-yl-ium; N-(2-hydroxyéthyl) pyridin-2-yl-ium; N-(2-hydroxyéthyl)pyridin-3-yl-ium; N-(2-hydroxyéthyl)pyridin-4-yl-ium; pyridin-1-yl-ium; trialkyl(C₁-C₄)ammonium-N-yl, 1-méthylpipéridinium-1-yl et 1,4-diméthylpipérazinium-1-yl.

Comme indiqué précédemment, la composition de teinture d'oxydation contenant le ou les composés de formule (I) conforme à l'invention permet d'obtenir des colorations puissantes dans des nuances allant du rouge au bleu et présentant de plus une ténacité remarquable aux différents traitements que peuvent subir les fibres kératiniques. Ces propriétés sont particulièrement remarquables notamment en ce qui concerne la résistance des colorations obtenues vis à vis de l'action de la lumière, des intempéries, des lavages, de l'ondulation permanente et de la transpiration.

Parmi les composés de formule (I) ci-dessus, on peut tout particulièrement citer :
- le chlorure de 3-[(3-hydroxy-4-méthanesulfonylamino-phénylcarbamoyl)-méthyl]-1-méthyl-3H-imidazol-1-ium ;
- le chlorure de 3-[(3-hydroxy-4-méthanesulfonylamino-6-méthoxy-phénylcarbamoyl)-méthyl]-1-méthyl-3H-imidazol-1-ium ;
- le chlorure de 1-[(3-hydroxy-4-méthanesulfonylamino-phénylcarbamoyl)-méthyl]-pyridinium ;
- le chlorure de 1-[(3-hydroxy-4-méthanesulfonylamino-6-chloro-phénylcarbamoyl)-méthyl]-pyridinium ;
- le chlorure de 1-[(3-hydroxy-4-méthanesulfonylamino-6-méthoxyphénylcarbamoyl)-méthyl]-pyridinium ;
- le chlorure de 1-[3-(2-hydroxy-6-amino-phénylcarbamoyl)-méthyl]-pyridinium ;
- le chlorure de 1-[(3-hydroxy-4-méthanesulfonylamino-phénylcarbamoyl)-méthyl]-1,4-diméthyl-pipérazin-1-ium ;
- le chlorure de 1-[(3-hydroxy-4-méthanesulfonylamino-6-chlorophénylcarbamoyl)-méthyl]-1,4-diméthyl-pipérazin-1-ium ;
- le chlorure de 1-[(3-hydroxy-4-méthanesulfonylamino-6-méthoxyphénylcarbamoyl)-méthyl]-1,4-diméthyl-pipérazin-1-ium ;
et leurs sels d'addition avec un acide.

Les composés de formule (I) conforme à l'invention peuvent être préparés selon des méthodes bien connues de l'état de la technique et décrites par exemple dans les demandes de brevet ou brevets JP59046645, JP 59039859, JP02072150, JP62108859, DE4238233, EP567172, DE2906526, DE2156480.

Un autre objet de l'invention est l'utilisation des composés de formules (I) conformes à l'invention à titre de coupleur pour la teinture d'oxydation des fibres kératiniques, et en particulier des fibres kératiniques humaines telles que les cheveux.

L'invention a également pour objet une composition pour la teinture d'oxydation des fibres kératiniques et en particulier des fibres kératiniques humaines telles que les cheveux, caractérisée par le fait qu'elle comprend, dans un milieu approprié pour la teinture, au moins un composé de formule (I) conforme à l'invention et au moins une base d'oxydation

Le ou les composés de formule (I) conformes à l'invention et/ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

La nature de la ou des bases d'oxydation pouvant être utilisées dans la composition tinctoriale conforme à l'invention n'est pas critique. Elles sont de préférence choisies parmi les bases d'oxydation classiquement utilisées en teinture d'oxydation et parmi lesquelles on peut notamment citer les paraphénylènediamines, les bis-phénylalkylènediamines, les para-aminophénols, les ortho-aminophénois et les bases hétérocycliques.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphényiènediamine, la N-(4'-aminophényl) paraphényténediamine, la N-phényl paraphénylénediamine, la 2-β-hydrdxyéthyloxy paraphénylènediamine, la 2-β-acétylaminoéthyloxy paraphénylènediamine, la N-(β-méthoxyéthyl)paraphénylènediamine, et leurs sels d'addition avec un acide.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénytènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophénjrl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ,ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazoto-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 9,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Selon l'invention, les compositions tinctoriales renfermant une ou plusieurs paraphénylènediamines et/ou une ou plusieurs bases d'oxydation hétérocycliques sont particulièrement préférées.

La ou les bases d'oxydation représentent de préférence de 0,0005 à 12 % en poids environ du poids total de la composition tinctoriale, et encore plus préférentiellement de 0.005 à 6 % en poids environ de ce poids.

La composition tinctoriale conforme à l'invention peut également renfermer, en plus du ou des composés de formule (I) ci-dessus, un ou plusieurs coupleurs additionnels pouvant être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indofiniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole; le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs additionnels représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (composés de formule (I), bases d'oxydation et coupleurs additionnels) sont notamment choisis parmi les chlorhydrates, les bromhydrates, les sulfates, les citrates, les succinates, les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 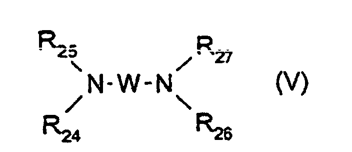 laquelle W est un reste propylène éventuellement substitué par un groupement hydroxyle ou un radical alkyle en C₁-C₆ ; R₂₄, R₂₅, R₂₆ et R₂₇, identiques ou différents, représentent un atome d'hydrogène, un radical alkyle en C₁-C₆ ou hydroxyalkyle en C₁-C₆.

Les compositions de teinture d'oxydation conformes à l'invention peuvent également renfermer au moins un colorant direct, notamment pour modifier les nuances ou les enrichir en reflets.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à ,50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, et les enzymes telles que les peroxydases, les laccases, les tyrosynases et les oxydo-réductases parmi lesquelles on peut en particulier mentionner les pyranose oxydases, les glucose oxydases, les glycérol oxydases, les lactates oxydases, les pyruvate oxydases, et les uricases.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcatinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention.

### EXEMPLE DE PREPARATION

### EXEMPLE DE PREPARATION 1 : Synthèse du chlorure de 3-[(3-hydroxy-4-méthanesulfonylamino-phénylcarbamoyl)-méthyl]-1-méthyl-3H-imidazol-1-ium.

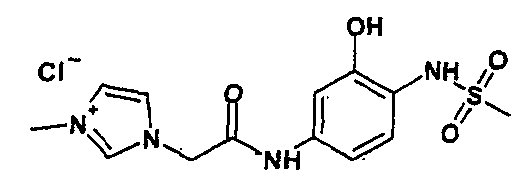

### a) Préparation du chlorhydrate de N-(4-amino-2-hydroxy-phényl)-méthanesulfonamide

12 g de N-(2-Hydroxy-4-nitro-phényl)-méthanesulfonamide (51 mmoles, préparé selon *Liebigs Ann. Chem.* **1994,** 269) dans 800 ml de méthanol ont été réduits sous hydrogène (18 bars) à une température de 40-44°C en 6 heures, en utilisant 2 g de palladium sur charbon (à 5%, 50% humide) comme catalyseur.
La solution filtrée a été versée sur 10 ml d'une solution méthanolique d'acide chlorhydrique (5,8 moles/l), puis concentrée à sec. La poudre obtenue a été lavée deux fois à l'éther diisopropylique et séchée sous vide jusqu'à poids constant pour donner 11,4 g de chlorhydrate de N-(4-amino-2-hydroxy-phényl)-méthanesulfonamide sous la forme d'une poudre beige avec un rendement de 92%.

### b) Préparation du 2-chloro-N-(3-hydroxy-4-méthanesulfonylamino-phényl)-acétamide

A une suspension de 5,5 g de chlorhydrate de N-(4-amino-2-hydroxy-phényl)-méthanesulfonamide (23 mmole) obtenu ci-dessus à l'étape précédente et de 4,6 g de carbonate de calcium (46 mmoles) dans 150 ml de dioxane, sous agitation et sous atmosphère inerte, a été ajouté goutte à goutte 1,85 ml de chlorure de chloroacétyle. Le milieu réactionnel a été agité à 40°C pendant 5 heures, refroidi à 15°C, filtré sur verre fritté et les sels minéraux ont été rincés deux fois au dioxane. Les phases organiques combinées ont été concentrées, reprises dans 10 ml de dioxane et versées sur de l'eau glacée. Le précipité formé a été essoré, puis repris dans du méthanol. La suspension obtenue a été filtrée sur célite et concentrée à sec. La poudre obtenue a été lavée au dichlorométhane et séchée sous vide jusqu'à poids constant pour donner 3,4 g de 2-chloro-N-(3-hydroxy-4-méthanesulfonylamino-phényl)-acétamide sous la forme d'une poudre beige avec un rendement de 53%.

### c) Préparation du chlorure de 3-[(3-hydroxy-4-méthanesulfonylaminophénylcarbamoyl)-méthyl]-1-méthyl-3H-imidazol-1-ium

Une solution de 2-chioro-N-(3-hydroxy-4-méthanesulfonylamino-phényl)-acétamide (2 g, 7,1 mmoles) obtenu ci-dessus à l'étape précédente, et de N-méthyl-imidazole (0,6 ml, 7,1 mmoles) dans 40 ml de dioxane a été chauffée au reflux pendant 8 heures. Le précipité formé a été essoré, lavé deux fois au dioxane et séché sous vide jusqu'à poids constant pour donner 1,97 g de chlorure de 3-[(3-hydroxy-4-méthanesulfonylamino-phénylcarbamoyl)-méthyl]-1-méthyl-3H-imidazol-1-ium sous la forme d'une poudre beige fondant à 205°C, avec un rendement de 77%. L'analyse en spectroscopie de masse cation : m/z = 325 du produit obtenu était conforme à celle du produit attendu.

### EXEMPLES DE TEINTURE

### EXEMPLES DE TEINTURE 1 à 4 EN MILIEU ALCALIN

On a préparé les compositions tinctoriales suivantes (teneurs en grammes) :

| **EXEMPLE** | **1** | **2** | **3** | **4** |
|---|---|---|---|---|
| Chlorure de 3-[(3-hydroxy-4-méthanesulfonylamino-phénylcarbamoyl)-méthyl]-1-méthyl-3H-imidazol-1-ium (composé de formule (I)) | 1,087 | 1,087 | 1,087 | 1,087 |
| Paraphénylènediamine (base d'oxydation) | 0,324 | - | - | - |
| 4,5-diamino 1-éthyl 3-méthyl pyrazole, 2HCl (base d'oxydation) | - | 0,639 | - | - |
| Pyrazolo-[1,5-a]-pyrimidine-3,7-diamine, 2HCl (base d'oxydation) | - | - | 0,666 | - |
| N,N-bis hydroxyéthyl paraphénytènediamine, 2HCl (base d'oxydation) | - | - | - | 0,882 |
| Support de teinture commun n°1 | (*) | (*) | (*) | (*) |
| Eau déminéralisée q.s.p. | 100 g | 100 g | 100 g | 100 g |

| | | | | |
|---|---|---|---|---|
| (*) Support de teinture commun n° 1 : | | | | |

- Alcool benzylique 2,0 g
- Polyéthylène glycol à 6 moles d'oxyde d'éthylène 3,0 g
- Ethanol à 96° 20,0 g
- Alkyl (C₈-C₁₀) polyglucoside en solution aqueuse à 60 % de matière active (M.A.), tamponnée par du citrate d'ammonium, vendu sous la dénomination ORAMIX CG 110 ® par la société SEPPIC 6,0 g
- Ammoniaque à 20% de NH₃ 10,0 g
- Métabisulfite de sodium à 35% de matière active 0,228 g
- Sel pentasodique de l'acide dléthylènetriaminopentacétique 1,1 g

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris naturels à 90 % de blancs pendant 30 minutes. Les mèches ont ensuite été rincées, lavées avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| 1 | 10 ± 0,2 | Châtain cendré |
| 2 | 10 ± 0,2 | Blond foncé légèrement violacé |
| 3 | 10 ± 0,2 | Châtain violine irisé |
| 4 | 10 ± 0,2 | Bleu |

## Revendications

1. Composés de formule (I) suivante, et leurs sels d'addition avec un acide : 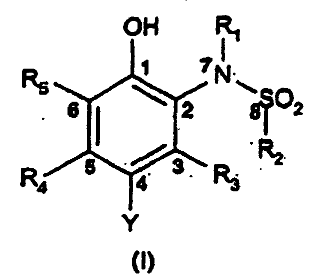 dans laquelle :
- R₁ représente un atome d'hydrogène ;
- R₂ représente un radical méthyle, éthyle ou diméthylamino ;
- R₃ représente un atome d'hydrogène ou un radical méthyle ;
- R₄ représente -NH-E-D₃, -NH(CO)-D₃, -NH(CO)-E-D₃, -NH(CO)O-E-D₃, -NH(CO)NH-E-D₃, ou -NH(SO₂)-E₃-D₃, ;
- R₅ représente un atome d'hydrogène, de chlore ou de fluor, ou un groupement méthyle, méthoxy, ou méthylamino ;
- Y représente un atome d'hydrogène ou de chlore, ou un groupement méthoxy, ou -OCH₂(CO)OCH₃ ;
- E représente un bras -(CH₂)_{q}-, q étant un nombre entier égal à 1 ou 2,
- D₃ est choisi parmi les groupements 3-méthylimidazolinium-1-yl, 3-(2-hydroxyéthyl)-imidazolinium-1-yl, 1,2,4-triazolium-1-yl; 1,2,4-triazolium-4-yl, N-alkyl(C₁-C₄)pyridin-2-yl-ium; N-alkyl(C₁-C₄)pyridin-3-yl-ium; N-alkyl(C₁-C₄)pyridin-4-yl-ium; N-(2-hydroxyéthyl) pyridin-2-yl-ium; N-(2-hydroxyéthyl)pyridin-3-yl-ium; N-(2-hydroxyéthyl)pyridin-4-yl-ium; pyridin-1-yl-ium; trialkyl(C₁-C₄)ammonium-N-yl, 1-méthylpipéridinium-1-yl et 1,4-diméthylpipérazinium-1-yl.

2. Composés selon la revendication 1,
**caractérisés par le fait qu'**lls sont choisis parmi :
- le chlorure de 3-[(3-hydroxy-4-méthanesulfonylamino-phénylcarbamoyl)-méthyl]-1-méthyl-3H-imidazol-1-ium ;
- le chlorure de 3-[(3-hydroxy-4-méthanesulfonylamino-6-méthoxy-phénylcarbamoyl)-méthyl]-1-méthyl-3H-imidazol-1-ium ;
- le chlorure de 1-[(3-hydroxy-4-méthanesulfonylamino-phénylcarbamoyl)-méthyl]-pyridinium ;
- le chlorure de 1-[(3-hydroxy-4-méthanesulfonylamino-6-chloro-phénylcarbamoyl)-méthyl]-pyridinium ;
- le chlorure de 1-[(3-hydroxy-4-méthanesulfonylamino-8-méthoxyphénylcarbamoyl)-méthyl]-pyridinium ;
- le chlorure de 1-[3-(2-hydroxy-6-amino-phénylcarbamoyl)-méthyl]-pyridinium ;
- le chlorure de 1-[(3-hydroxy-4-méthanesulfonylamino-phénylcarbamoyl)-méthyl]-1,4-diméthyl-pipérazin-1-ium ;
- le chlorure de 1-[(3-hydroxy-4-méthanesulfonylamino-6-chlorophénylcarbamoyl)-méthyl]-1,4-diméthyl-pipérazin-1-ium ;
- le chlorure de 1-[(3-hydroxy-4-méthanesulfonylamino-6-méthoxyphénylcarbamoyl)-méthyl]-1,4-diméthyl-pipérazin-1-ium ;
et leurs sels d'addition avec un acide.

3. Composés selon l'une quelconque des revendications précédentes, **caractérisés par** le falt que les sels d'addition avec un acide sont choisis parmi les chlorhydrates, les bromhydrates, les sulfates, les citrates, les succinates, les tartrates, les lactates et les acétates.

4. Utillsation des composés de formule (I) tels que définls à l'une quelconque des revendications 1 à 3, à titre de coupleur pour la teinture d'oxydation des fibres kératiniques.

5. Composition pour la teinture d'oxydation des fibres kératiniques, **caractérisée par le fait qu'**elle contient, dans un milleu approprié pour la teinture :
- au moins une base d'oxydation, et
- au moins un coupleur choisi parmi les composés de formule (I) tels que définis à l'une quelconque des revendications 1 à 3.

6. Composition selon la revendication 5, **caractérisée par le fait que** le ou les composés de formule (I) et/ou le ou leurs sels d'addition avec un acide représentent de 0,0005 à 12 % en poids du poids total de la composition tinctoriale.

7. Composition selon la revendication 5 ou 6, **caractérisée par le fait que** la ou les bases d'oxydation sont choisies parmi les paraphénylènediamines, les bis-phénylalkylènediamines, les para-aminophénols, les ortho-aminophénols et les bases hétérocycliques, et leurs sels d'addition avec un acide.

8. Composition selon l'une quelconque des revendications 5 à 7; **caractérisée par le fait que** la ou les bases d'oxydation représentent de 0,0005 à 12 % en poids du poids total de la composition tinctoriale.

9. Composition selon l'une quelconque des revendications 5 à 8, **caractérisée par le fait qu'**elle renferme en plus du ou des composés de formule (I) ci-dessus, un ou plusieurs coupleurs additionnels choisis parmi les métaphénylènediamines, les méta-aminophénois, les métadiphénois et les coupleurs hétérocycliques, et leurs sels d'addition avec un acide, et/ou un ou plusieurs colorants directs.

10. Composition selon la revendication 9, **caractérisée par le fait que** le ou les coupleurs additionnels représentent de 0,0001 à 10 % en poids du poids total de la composition tinctoriale.

11. Composition selon l'une quelconque des revendications 5 à 10, **caractérisée par le fait que** les sels d'addition avec un acide sont choisis parmi les chlorhydrates, les bromhydrates, tes sulfates, les citrates, les succinates, les tartrates, les lactates et les acétates.

12. Procédé de teinture d'oxydation des fibres kératiniques, **caractérisé par le fait que** l'on applique sur ces fibres au moins une composition tinctoriale telle que définie à l'une quelconque des revendications 5 à 11, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

13. Procédé selon la revendication 12, **caractérisé par le fait que** l'agent oxydant est choisi parml le peroxyde d'hydrogène, le peroxyde d'urée, les bromates de métaux alcallns, tes persels, et les enzymes.

14. Dispositif à plusieurs compartiments, ou "kit" de teinture à plusieurs compartiments, dont un premier compartiment renferme une composition tinctoriale telle que définie à l'une quelconque des revendications 5 à 11 et un second compartiment renferme une composition oxydante.

## Patentansprüche

1. Verbindungen der folgenden Formel (I) und deren Additionssalze mit einer Säure: 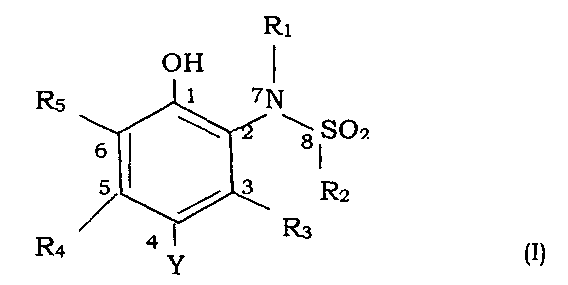 wobei in der Formel bedeuten:
· R₁ Wasserstoff;
· R₂ Methyl, Ethyl oder Dimethylamino;
· R₃ Wasserstoff oder Methyl;
· R₄ -NH-E-D₃, -NH(CO)-D₃, -NH(CO)-E-D₃, -NH(CO)O-E-D₃, -NH(CO)NH-E-D₃ oder -NH(SO₂)-E-D₃;
· R₅ Wasserstoff; Chlor, Fluor, Methyl, Methoxy oder Methylamino;
· Y Wasserstoff, Chlor, Methoxy oder -OCH₂(CO)OCH₃;
· E bedeutet (CH₂)_{q}, wobei q 1 oder 2 bedeutet;
· D₃ eine Gruppe, die ausgewählt ist unter: 3-Methylimidazolinium-1-yl, 3-(2-Hydroxyethyl)-imidazolinium-1-yl, 1,2,4-Triazolium-1-yl, 1,2,4-Triazolium-4-yl, N-C₁₋₄-Alkylpyridin-2-ylium, N-C₁₋₄-Alkyl-pyridin-3-ylium, N-C₁₋₄-Alkylpyridin-4-ylium, N-(2-Hydroxyethyl)-pyridin-4-ylium, N-(2-Hydroxyethyl)-pyridin-2-ylium, N-(2-Hydroxyethyl)-pyridin-3-ylium, N-(2-Hydroxyethyl)-pyridin-4-ylium, Pyridin-1-ylium, Trialkyl(C₁₋₄)ammonium-N-yl, 1-Methyl-piperidinium-1-yl und 1,4-Dimethyl-piperazinium-1-yl.

2. Verbindungen nach Anspruch 1, **dadurch gekennzeichnet, dass** sie unter den folgenden Verbindungen ausgewählt sind:
· 3-[(3-Hydroxy-4-methansulfonylamino-phenylcarbamoyl)-methyl]-1-methyl-3H-imidazol-1-iumchlorid;
· 3-[(3-Hydroxy-4-methansulfonylamino-6-methoxy-phenylcarbamoyl)-methyl]-1-methyl-3H-imidazol-1-iumchlorid;
· 1-[(3-Hydroxy-4-methansulfonylamino-phenylcarbamoyl)-methyl]-pyridiniumchlorid;
· 1-[(3-Hydroxy-4-methansulfonylamino-6-chlor-phenylcarbamoyl)-methyl]-pyridiniumchlorid;
· 1-[(3-Hydroxy-4-methansulfonylamino-6-methoxy-phenylcarbamoyl)-methyl]-pyridiniumchlorid;
· 1-[3-(2-Hydroxy-6-amino-phenylcarbamoyl)-methyl]-pyridiniumchlorid;
· 1-[(3-Hydroxy-4-methansulfonylamino-phenylcarbamoyl)-methyl]-1,4-dimethyl-piperzin-1-iumchlorid;
· 1-[(3-Hydroxy-4-methansulfonylamino-6-chlor-phenylcarbamoyl)-methyl]-1,4-dimethyl-piperzin-1-iumchlorid;
· 1-[(3-Hydroxy-4-methansulfonylamino-6-methoxy-phenylcarbamoyl)-methyl]-1,4-dimethyl-piperzin-1-iumchlorid; und
· deren Additionssalzen mit einer Säure.

3. Verbindungen nach einem der vorhergehenden Ansprüche, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

4. Verwendung von Verbindungen der Formel (I) nach einem der Ansprüche 1 bis 3 als Kuppler zum oxidativen Färben von Keratinfasern.

5. Zusammensetzung zum oxidativen Färben von Keratinfasern, **dadurch gekennzeichnet, dass** sie in einem zum Färben geeigneten Medium enthält:
- mindestens eine Oxidationsbase, und
- mindestens einen Kuppler, der unten den Verbindungen der Formel (I) nach einem der Ansprüche 1 bis 3 ausgewählt ist.

6. Zusammensetzung nach Anspruch 5, **dadurch gekennzeichnet, dass** die Verbindung(en) der Formel (I) und/oder ihr(e) Additionssalz(e) mit einer Säure 0,0005 bis 12 Gew.-% des Gesamtgewichts der Farbmittelzusammensetzung ausmachen.

7. Zusammensetzung nach Anspruch 5 oder 6, **dadurch gekennzeichnet, dass** die Oxidationsbase(n) unter den p-Phenylendiaminen, Bisphenylalkylendiaminen, p-Aminophenolen, o-Aminophenolen und den heterocyclischen Basen und ihren Additionssalzen mit einer Säure ausgewählt sind.

8. Zusammensetzung nach einem der Ansprüche 5 bis 7, **dadurch gekennzeichnet, dass** die Oxidationsbase(n) 0,0005 bis 12 Gew.-% des Gesamtgewichts der Farbmittelzusammensetzung ausmachen.

9. Zusammensetzung nach einem der Ansprüche 5 bis 8, **dadurch gekennzeichnet, dass** sie neben der oder den Verbindungen der Formel (I) ferner einen oder mehrere zusätzliche Kuppler, die unter den m-Phenylendiaminen, m-Aminophenolen, m-Dihydroxybenzolen und den heterocyclischen Kupplern ausgewählt sind, und deren Additionssalze mit einer Säure und/oder einen oder mehrere Direktfarbstoffe enthält.

10. Zusammensetzung nach Anspruch 9, **dadurch gekennzeichnet, dass** der oder die zusätzlichen Kuppler 0,0001 bis 10 Gew.-% des Gesamtgewichts der Farbmittelzusammensetzung ausmachen.

11. Zusammensetzung nach einem der Ansprüche 5 bis 10, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

12. Verfahren zum oxidativen Färben von Keratinfasern, **dadurch gekennzeichnet, dass** auf die Fasern mindestens eine Farbmittelzusammensetzung nach einem der Ansprüche 5 bis 11 aufgetragen wird und die Farbe bei einem sauren, neutralen oder alkalischen pH-Wert mit einem Oxidationsmittel entwickelt wird, das kurz vor der Anwendung zu der Farbmittelzusammensetzung gegeben wird oder das in einer oxidierenden Zusammensetzung vorliegt, die gleichzeitig oder getrennt davon anschließend aufgetragen wird.

13. Verfahren nach Anspruch 12, **dadurch gekennzeichnet, dass** das Oxidationsmittel unter Wasserstoffperoxid, Harnstoffperoxid, Alkalimetallbromaten, Salzen von Persäuren und Enzymen ausgewählt ist.

14. Vorrichtung mit mehreren Abteilungen oder "Kit" zum Färben mit mehreren Abteilungen, wobei eine erste Abteilung eine Farbmittelzusammensetzung nach einem der Ansprüche 5 bis 11 und eine zweite Abteilung eine oxidierende Zusammensetzung enthält.

## Claims

1. Compounds of formula (I) below, and the addition salts thereof with an acid: 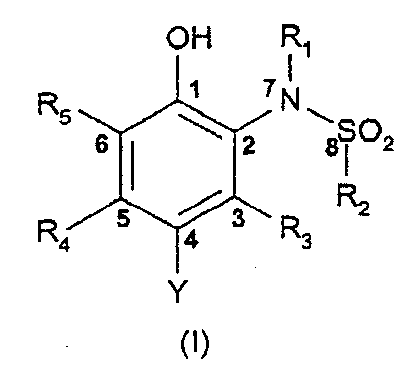 in which:
- R₁ represents a hydrogen atom;
- R₂ represents a methyl, ethyl or dimethylamino radical;
- R₃ represents a hydrogen atom or a methyl radical;
- R₄ represents -NH-E-D₃, -NH(CO)-D₃, -NH(CO)-E-D₃, -NH(CO)O-E-D₃, -NH(CO)NH-E-D₃ or -NH(SO₂)-E₃-D₃;
- R₅ represents a hydrogen, chlorine or fluorine atom or a methyl, methoxy or methylamino group;
- Y represents a hydrogen or chlorine atom or a methoxy or -OCH₂(CO)OCH₃ group;
- E represents an arm -(CH₂)_{q}-, q being an integer equal to 1 or 2,
- D₃ is chosen from 3-methylimidazolinium-1-yl, 3-(2-hydroxyethyl)imidazolinium-1-yl, 1,2,4-triazolium-1-yl, 1,2,4-triazolium-4-yl, N-(C₁-C₄)alkylpyrid-2-ylium, N-(C₁-C₄)alkylpyrid-3-ylium, N-(C₁-C₄)alkylpyrid-4-ylium, N-(2-hydroxyethyl)pyrid-2-ylium, N-(2-hydroxyethyl)-pyrid-3-ylium, N-(2-hydroxyethyl)pyrid-4-ylium, pyrid-1-ylium, tri (C₁-C₄)alkylammonium-N-yl, 1-methylpiperidinium-1-yl and 1,4-dimethylpiperazinium-1-yl groups.

2. Compounds according to Claim 1, **characterized in that** they are chosen from:
- 3-[(3-hydroxy-4-methanesulphonylaminophenylcarbamoyl)methyl]-1-methyl-3H-imidazol-1-ium chloride;
- 3-[(3-hydroxy-4-methanesulphonylamino-6-methoxyphenylcarbamoyl)methyl]-1-methyl-3H-imidazol-1-ium chloride;
- 1-[(3-hydroxy-4-methanesulphonylaminophenylcarbamoyl)methyl]-pyridinium chloride;
- 1-[(3-hydroxy-4-methanesulphonylamino-6-chlorophenylcarbamoyl)methyl]pyridinium chloride;
- 1-[(3-hydroxy-4-methanesulphonylamino-6-methoxyphenylcarbamoyl)methyl]pyridinium chloride;
- 1-[3-(2-hydroxy-6-aminophenylcarbamoyl)methyl]pyridinium chloride;
- 1-[(3-hydroxy-4-methanesulphonylaminophenylcarbamoyl)methyl]-1,4-dimethylpiperazin-1-ium chloride;
- 1-[(3-hydroxy-4-methanesulphonylamino-6-chlorophenylcarbamoyl)methyl]-1,4-dimethylpiperazin-1-ium chloride;
- 1-[(3-hydroxy-4-methanesulphonylamino-6-methoxyphenylcarbamoyl)methyl]-2,4-dimethylpiperazin-1-ium chloride;
and the addition salts thereof with an acid.

3. Compounds according to any one of the preceding claims, **characterized in that** the addition salts with an acid are chosen from the hydrochlorides, hydrobromides, sulphates, citrates, succinates, tartrates, lactates and acetates.

4. Use of the compounds of formula (I) as defined in any one of Claims 1 to 3, as couplers for the oxidation dyeing of keratin fibres.

5. Composition for the oxidation dyeing of keratin fibres, **characterized in that** it contains, in a medium which is suitable for dyeing:
- at least one oxidation base, and
- at least one coupler chosen from the compounds of formula (I) as defined in any one of Claims 1 to 3.

6. Composition according to Claim 5, **characterized in that** the compound(s) of formula (I) and/or the addition salt(s) thereof with an acid represent(s) from 0.0005% to 12% by weight relative to the total weight of the dye composition.

7. Composition according to Claim 5 or 6, **characterized in that** the oxidation base(s) is(are) chosen from para-phenylenediamines, bis(phenyl)alkylenediamines, para-aminophenols, ortho-aminophenols and heterocyclic bases, and the addition salts thereof with an acid.

8. Composition according to any one of Claims 5 to 7, **characterized in that** the oxidation base(s) represent(s) from 0.0005% to 12% by weight relative to the total weight of the dye composition.

9. Composition according to any one of Claims 5 to 8, **characterized in that**, in addition to the compound(s) of formula (I) above, it contains one or more additional coupler(s) chosen from metaphenylenediamines, meta-aminophenols, meta-diphenols and heterocyclic couplers, and the addition salts thereof with an acid, and/or one or more direct dyes.

10. Composition according to Claim 9, **characterized in that** the additional coupler(s) represent(s) from 0.0001% to 10% by weight relative to the total weight of the dye composition.

11. Composition according to any one of Claims 5 to 10, **characterized in that** the addition salts with an acid are chosen from the hydrochlorides, hydrobromides, sulphates, citrates, succinates, tartrates, lactates and acetates.

12. Process for the oxidation dyeing of keratin fibres, **characterized in that** at least one dye composition as defined in any one of Claims 5 to 11 is applied to these fibres, and **in that** the colour is developed at acidic, neutral or alkaline pH using an oxidizing agent which is added to the dye composition just at the time of use, or which is present in an oxidizing composition applied simultaneously or sequentially in a separate manner.

13. Process according to Claim 12, **characterized in that** the oxidizing agent is chosen from hydrogen peroxide, urea peroxide, alkali metal bromates, persalts and enzymes.

14. Multi-compartment device or multi-compartment dyeing kit, a first compartment of which contains a dye composition as defined in any one of Claims 5 to 11, and a second compartment of which contains an oxidizing composition.
